# EUROPEAN PATENT SPECIFICATION

(11) **EP 0 426 300 B1**
(45) Date of publication and mention of the grant of the patent: **03.06.1998**
(21) Application number: 90310671.4
(22) Date of filing: 28.09.1990
(51) Int. Cl.: G01N 33/58, B01J 13/00

(54) **Method of producing a reagent containing a narrow distribution of colloidal particles of a selected size and the use thereof**
Verfahren zur Herstellung eines Reagens, welches eine enge Verteilung kolloidaler Teilchen mit selektierter Grösse enthält und Verwendung desselben
Méthode de production d'un réactif contenant une distribution étroite de particules colloidales de taille sélectionnée et l'utilisation de celui-ci

(30) Priority: 29.09.1989 US 415184
(43) Date of publication of application: 08.05.1991
(73) Proprietor: ORTHO DIAGNOSTIC SYSTEMS INC., Raritan, New Jersey 08869-0602 (US)
(72) Inventor: Schutt, Ernest G., Long Valley, NJ 07853 (US); Le, Anh V., Somerville, NJ 08876 (US); Cennerazzo, Michael J., Weehawken, NJ 07087 (US); Nemore, Robert E., Easton, PA 18042 (US)
(74) Representative: Mercer, Christopher Paul

(56) References cited:
- EP-A- 0 310 872
- US-A- 4 313 734
- Weiser, H.B., "The Colloidal Elements" in Inorganic Colloid Chemistry, J. Wiley & Sons (1933) pages 1-107.
- DATABASE WPIL, accession no. 89-034892 [05], Derwent Publications Ltd, London, GB; & JP-A-63 307 208 (CHIYODA CHEM. ENG. CO.)

## Description

### Field of the Invention

The invention relates to a method for the production of reagents having particles of a selected size and narrow distribution for use in electron microscopy, photonic and light microscopy, and immunoassay techniques.

### Background of the Invention

Colloidal solutions or sols can be formed by condensation processes. In the condensation method, the colloid is formed from micromolecular units, the so called "nuclei" which grow into particles of varying sizes depending on the specific conditions employed. This condensation process may be regarded as a process of crystallization since the colloid particles have a crystalline structure. Conventionally, a reducing agent is added to a metal salt forming metallic particles. As this reaction occurs, minute crystallization centers or metallic nuclei are formed. The process of nuclei generation determines the number of nuclei or seed particles produced. As with other crystallization processes, the faster the metallic particles are generated,the more nuclei are formed, and therefore the smaller the final particle diameter.

The nuclei generation process is generally highly dependent on temperature, instantaneous pH of a rapidly changing solution, chelation by the reducing agent, reagent concentration and reagent purity. In addition, nuclei are generated throughout the reduction process leading to differing growth times and a diverse population of particle diameters.

The most frequently employed method for preparing gold hydrosols is the reduction of chloroauric acid with a suitable reducing agent. A number of reducing agents can be used including formaldehyde, hydrogen peroxide, phosphorus, and substituted ammonias such as hydroxylamine and hydrazine. Methods of production of gold sols are generally discussed in: Roth, J. "The Colloidal Gold Marker System for Light and Electron Microscopic Cytochemistry", in Bullock, G.R. and Petrusz, P. (eds.), Techniques in Immunocytochemistry 2, pp. 217-284; Horisberger, M. "Colloidal Gold: A Cytochemical Marker For Light and Fluorescent Microscopy and For Transmission and Scanning Electron Microscopy", SEM, 11:9-31 (1981); and Weiser, H.B., "The Colloidal Elements", in Inorganic Colloid Chemistry, J. Wiley & Sons (1933), pp. 1-107. In the absence of added nuclei, the size of the primary gold particles in a sol will be determined, for a given concentration of reactants, by the velocity with which nuclei form spontaneously and the velocity with which the particles grow from the spontaneously formed nuclei.

The spontaneous formation of nuclei can be greatly retarded and the growth of particles promoted using specific reducing agents, such as hydroxylamine, hydrazine and their salts. For example, addition of a dilute aqueous solution of hydroxylamine to a chloroauric acid-potassium carbonate mixture will result in a suspension of relatively large gold particles because hydroxylamine retards the spontaneous formation of nuclei and favors the rapid growth of particles.

To produce gold sol without the addition of nuclei, a first reducing agent such as potassium thiocyanate is added to a solution of chloroauric acid to bring about the formation of nuclei. A second reducing agent, such as hydroxylamine is then added which causes growth on the nuclei already present but suppresses the further formation of nuclei. See, Weiser, page 30, lines 16-23.

A series of sols with particles of widely varying size can be prepared by the addition of nuclei in varying amounts when adding the reducing agent. Therefore, it is possible to determine conditions for forming sols of like metal content but with varying particle size. Generally, the greater the number of added nuclei, the smaller the particles formed. To obtain gold sol with a graded number of particles of definite size, a small amount of nuclei can be added before adding a reducing agent. To prevent spontaneous formation of nuclei, Weiser teaches adding potassium ferrocyanide before reduction. See Weiser page 30, lines 24-40. Weiser teaches making gold nuclei by reduction with phosphorous at page 39, lines 9-16. At page 40, first full paragraph, Weiser in the course of discussing gold sols with varying particle size obtained by reduction with hydroxylamine in the presence of nuclei concludes that it is obvious that, the greater the number of added nuclei, the smaller the particles and the redder and clearer the sol, as a means of emphasizing the importance of the spontaneous formation of nuclei on the size of particles in a sol formed without the addition of nuclear liquid. However, Weiser does not anticipate the production of gold particles of a selected size range and narrow distribution, in any reproducible manner, nor does he teach any method for producing gold nuclei of any selected size to be used as seed nuclei to produce gold particles of a selected size and narrow distribution, nor does he comment upon the coatability or utility of such particles.

Gold particles ranging in size from 2-140 nm are readily produced by a variety of methods. See Geoghegon, W.D. "Immunoassays at the Microscopic Level: Solid-Phase Colloidal Gold Methods", J. Clin Immunoassay 11(1):11-23 (1988). Using trisodium citrate as a reductant, particle diameter has been found to be a function of the quantity of citrate added. See Frens, G. "Controlled Nucleation for the Regulation of the Particle Size in Monodisperse Gold Suspensions", Nature Physical Sci. 241:20-22 (1973). Particle size may be varied over an approximate size range of 15-140 nm by varying the amount of citrate added. The eventual size of the particles is governed by the number of nuclei which form and grow into particles. Particles in the 2-20 nm range can be produced using white phosphorus, ascorbic acid, or a mixture of tannic acid and citrate as the reductant.

Colloidal gold can be used as a particulate marker for the detection and localization of target molecules by various modes of microscopy using both direct and indirect labelling approaches. Under appropriate conditions, colloidal gold will bind macromolecules by non-covalent electrostatic adsorption with little change in the specific activity of the bound macromolecule. Interaction is influenced by ionic concentration, pH conditions in correlation with protein isoelectric points and protein stabilizing levels.

Under appropriate conditions, metal sol particles can be labelled with a variety of macromolecules, including polysaccharides, glycoproteins, proteins, lectins and antibodies. Whenever the term "metal sol particles" is used in this application, this is understood to mean particles of a sol consisting of a metal or transition metal, a metal or transition metal compound or polymer nuclei coated with a metal or transition metal or a metal or transition metal compound. The metal sols may be of metals or transition metals or compounds thereof such as oxides, hydroxides, and salts or of polymer nuclei coated with metals or transition metals or compounds thereof. Examples include platinum, gold, silver, iron, copper, selenium, chromium, vanadium, titanium, and manganese. In addition, it is recognized that a metal sol produced in accordance with the teachings of Applicants' invention may be converted to a suspension of insoluble metal salts, sulphides, oxides, hydroxides or similar compounds. In general, all metals, transition metals or compounds thereof, which may be readily demonstrated by means of techniques well known in the art are within the scope of Applicants' invention. For a general discussion concerning gold particle labelling techniques, see Horisberger, M. "Colloidal Gold: A Cytochemical Marker For Light and Fluorescent Microscopy and For Transmission and Scanning Electron Microscopy", SEM, 11:9-31 (1981). In most cases there is little change in the bioactivity of the adsorbed molecules. Generally, these probes acquire the specific activity of the adsorbed macromolecule and their stability upon storage is good. However, when gold particles are labelled with proteins, full stabilization against coagulation by electrolytes is not always observed, especially with larger size markers. A number of stabilizing agents including polyvinylpyrrolidone, poly-L-lysine, poly-L-proline, polyethylene glycols (PEG), and Carbowax have been suggested. For example, see Horisberger, page 30, second column. Bovine serum albumin (BSA) has also been suggested as a protecting protein but is not believed to be a good choice by Horisberger because it binds to numerous physiological anions. See, Horisberger, page 12, lines 58-63.

A procedure using non-fat dry milk as a protein-nucleic acid source for blocking non-specific reactions, as an incubation medium, and for subsequent washing to remove unreacted reagents for analysis of proteins (Western blotting) and DNA (Southern blotting) transferred to nitrocellulose for reaction with antibodies or nucleic acid probes is described in Johnson, D.A., Gautsch, J.W. Sportsman, J.R. and Elder, J.H. "Improved Technique Utilizing Non-Fat Dry Milk for Analysis of Proteins and Nucleic Acids Transferred to Nitrocellulose", Gene Anal. Techn. 1:3-8 (1984). The use of non-fat dry milk as a replacement for bovine serum albumin or gelatin as reagents used to prevent non-specific binding is disclosed for methods to facilitate the transfer of proteins and nucleic acids to nitrocellulose.

As probes, gold particles are particularly interesting because their electron dense properties allow detection by transmission electron microscopy (TEM), their capability of strong emission of secondary electrons allows visualization by scanning electron microscopy (SEM), their characteristic X-ray signals allow identification of gold markers on cell surfaces. In addition, gold probes are also useful in fluorescent microscopy by labelling gold particles with fluorescent molecules. Gold particles bound to a cell surface appear as an orange-red coating and are therefore useful in photonic microscopy and in macroscopic observations. The advantages of gold probes are discussed generally in Horisberger, M. "Colloidal Gold: A Cytochemical Marker For Light and Fluorescent Microscopy and For Transmission and Scanning Electron Microscopy", SEM, 11:9-31 (1981); Goodman, S.L. et al. "A Review of the Colloidal Gold Marker System", SEM 11:133-146 (1980). A bibliography of gold probe labelling studies is provided by Goodman, at page 139.

It has been shown that it is possible to select and adsorb a specific substance to colloidal gold to optimize its use as a tracer for electron microscopy (EM). It has been suggested that an ideal tracer substance should be available in a wide range of uniform sizes with an electron scattering core surrounded by a coat that could be varied as needed. See Geoghegon, W.D. "Immunoassays at the Microscopic Level: Solid-Phase Colloidal Gold Methods", J. Clin Immunoassay 11(1) 11-23 (1988). The gold is stabilized by a variety of substances, including polypeptides, polynucleotides, polysaccharides and organic polymers.

There are a number of immunoassays employing colloidal gold. The presence of a reactive protein on a probe can be demonstrated and quantitated by direct and indirect radioactive binding assays and agglutination assays. See Goodman, S.L. et al. "A Review of the Colloidal Gold Marker System", SEM 11:133-146 (1980).

To make a gold probe, the following basic steps are followed: a protein solution and colloidal gold are pH adjusted to optimize protein adsorption, the minimal protecting amount of protein is determined, the appropriate amount of protein is mixed with the colloidal gold and a secondary stabilizer added. The gold probe can then be purified and its concentration adjusted to a predetermined optical density. Using these general principles, ligands can be bound to gold probes for use in immunoassays. The Janssen manual entitled "Colloidal Gold Sols for Macromolecular Labelling" (1985) discloses basic probe construction techniques.

U.S. Patent No. 4,313,734 (Leuvering) discloses a metal sol particle immunoassay. Leuvering teaches the preparation of a gold sol by reducing chloroauric acid with trisodium citrate to produce gold particles with diameters of 45-70 nm. The particles are then labelled with a rabbit anti-HPL serum by adjusting the sol to pH 7.0 with potassium carbonate and then adding a rabbit anti-HPL immunoglobulin solution. The coated gold particles can then be used for determination of HPL by colorimetry or atomic adsorption spectrophotometry. Leuvering also discloses the visual detection of hepatitis Bs antigen (HBsAg) by means of a gold particle sheep anti-HBs immunoglobulin conjugate. In this method the gold sol and the gold-immunoglobulin conjugate are prepared as before with the exception that a diluted solution of the sheep anti-HBs immunoglobulin solution was used instead of the rabbit anti-HPL immunoglobulin solution. Leuvering also discloses the determination of HCG with the aid of a gold particle-anti-HCG conjugate in an agglutination test. The gold sol and gold particle anti-HCG conjugate are prepared using the previously described methods. A competitive receptor assay for HCG is also disclosed. A gold dispersion consisting of particles having a diameter between 6-15 nm is prepared by adding sodium citrate to a boiling solution of chloroauric acid. A dialyzed HCG solution was added to the gold dispersion. The mixture was stabilized with Carbowax. A sandwich assay for HCG using an insolubilized HCG receptor and the gold particle-anti HCG conjugate is then performed. In patent Abstracts of Japan, Vol. 13, No 143, JP-A-63 307 208 is described detailing a method for producing dispensed crystalline fine platinum particles by blowing a reducing gas into an aqueous chloroplatinic acid solution.

### Summary of the Invention

The seeded sol process of the present invention employs a reducing agent from a family of compounds that retard the spontaneous formation of nuclei. For example, hydroxylamine and gold chloride can be mixed for extended periods of time with no visible reaction, but the addition of nuclei or seeds to the mixture allows gold to deposit on the seeds, producing larger gold particles. It was surprisingly found that the final diameter of metal sol particles can be determined almost exclusively by the number of seeds added. The reaction is nearly independent of (1) temperature; (2) small variations in concentration of the reducing agent; (3) pH of reactants; (4) small variations in metal salt concentration. It has been found that using this method, the final metal sol particle diameter is inversely proportional to the cube root of added seed volume. Therefore, conditions to make particles of a specific diameter can be reliably calculated and small variations in seed volume or the metal salt concentration do not significantly affect particle diameter. As all the seeds are exposed to growth at the same time in the present invention, unlike the gradual nucleation of other processes, the product particles are also significantly more uniform.

The present invention provides a method for producing metallic sol particles of a selected size range which comprises:
(a) reducing a solution containing a metal and a stabilising agent with a first reducing agent to form metallic nuclei; and
(b) mixing the metallic nuclei formed in step (a) with a solution containing said metal and a second reducing agent to form metallic sol particles,
wherein:
said stabilising agent is citrate, malate, citric acid, malic acid, oxalate, oxalic acid, aspartate, aspartic acid, glutamate or glutamic acid;
said first reducing agent and said stabilising agent are selected so that in step (b) they will not generate spontaneous nuclei and will not retard growth of nuclei in the subsequent particle growth step; and
said second reducing agent retards spontaneous enucleation.

Preferred features of the present invention are detailed in the dependant claims.

The sol produced according to Applicant's method is then coated with a protein. As protective proteins it is possible to use antigens, antibodies or polypeptide fragments thereof which are still immunologically active. Furthermore, it is possible to envisage haptens attached to macromolecules (e.g. proteins, polysaccharides, lectins or surfactants) which during the pertinent immunoassay do not give rise to any interfering reaction with other components.

The protein amount and coating pH are determined by a salt flocculation study. General procedures for salt flocculation determination of pH and protein coating concentration are summarized in the Janssen manual entitled "Colloidal Gold Sols for Macromolecular Labelling" (1985) which accompanies raw gold sol which may be purchased from that company. The required amount of protein is diluted in an appropriate coating buffer which may contain bovine serum albumin (BSA). When the coating protein is placed in a buffer containing BSA in large excess, the BSA acts as a scavenger for the hydroxylamine and its reaction byproducts but does not interfere with the binding of the coating protein to the metal sol particle surface, preventing any loss of activity or stability due to hydroxylamine. Hydroxylamine and its reaction byproducts can also be inactivated by adjusting the pH, a combination of pH and BSA, or use of chemical agents.

The metal sol particle-protein conjugate is then post-coated with delipidated milk, most preferably using a non-fat dry milk solution to stabilize the reagent. Applicants surprisingly found that non-fat dry milk is an effective post-coater to stabilize the metal sol coated particles. The resulting reagent can then be stored in an appropriate storage buffer, preferably containing BSA and a low molecular weight sugar such as mannitol. Applicants have found the reagent prepared by this method to be particularly useful in a rapid dot blot assay. In this assay, the antibody is slotted onto nitrocellulose. A mixture containing the sample to be assayed and a gold reagent prepared according to Applicants' method is then filtered through. If the gold is retained, a red or purple dot is visible. This assay can be used to detect the presence of mouse IgG adsorbed onto the gold particles, for example.

Applicants have also found the reagent to be useful in an immunoassay system developed by Applicants which utilizes scattered total internal reflectance (STIR) of light as a measure of the presence of particular ligands to be determined in an aqueous solution. As used herein, the term "ligand" includes immunoglobulins, whether monoclonal or polyclonal, and their respective binding partners, which may be haptens, antigens, or other analytes. Metal sol particles can be used as a label for a solution phase immunologically active component in Applicants' STIR method. It was surprisingly found that the combination of STIR with colloidal gold results in an extremely efficient and sensitive homogenous assay system. STIR assays for TSH, T₄, CEA, AFP, HCG, anti-HIV, HBsAg, HBc, theophylline and digoxin are performed according to this method.

The method in accordance with the present invention is particularly suitable for the qualitative and/or quantitative determination of an immunochemically reactive component, such as a hapten, antigen, or antibody present in an aqueous test medium, but can also be employed for the histological or cytological determination of such components.

For this reason the invention similarly relates to the new immunological reagents consisting of an aqueous dispersion of metal sol particles to which either directly or indirectly an immunochemically reactive component has been attached.

The present invention provides a kit for performing an immunoassay comprising: coated or post-coated sol particles produced by the method of the present invention; and an assay buffer.

These and other objects of the present invention will become apparent from the following, more detailed description and is illustrated in its specific embodiment in the accompanying drawings.

### Brief Description of the Drawings

The subject of the instant invention for which protection is sought is presented as claims at the conclusion of the written description of the invention set forth herein. The description sets forth the manner and process for making and using the invention and the best mode contemplated therefor, and the accompanying drawings form part of the description for illustrating the practice of the invention.

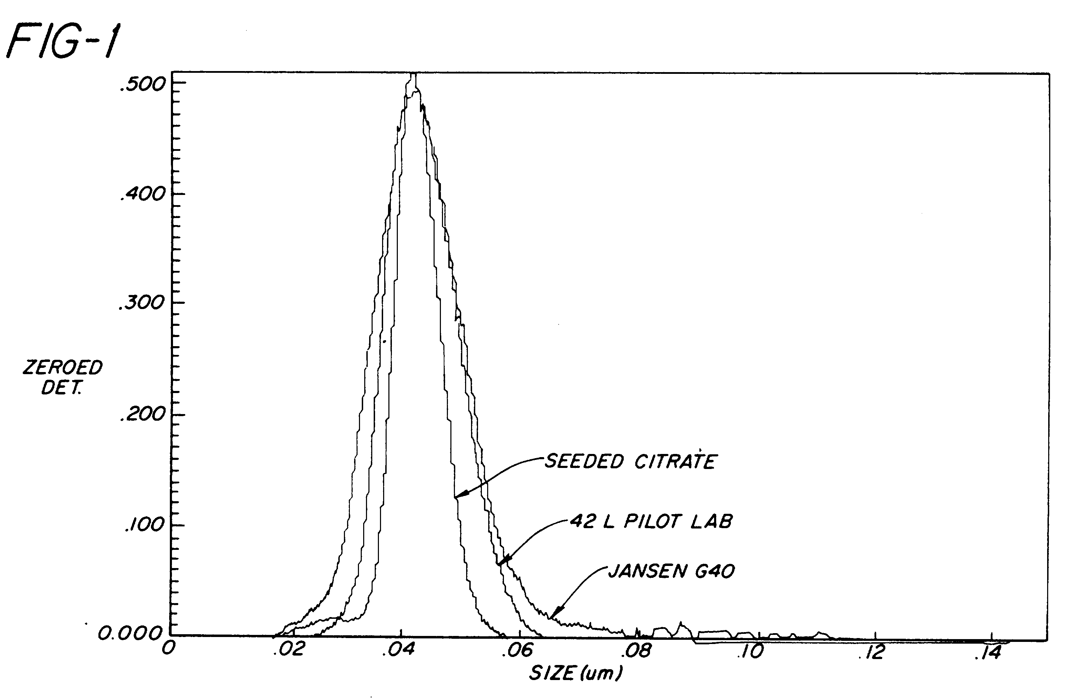

Figure 1 is a graph illustrating the narrow distribution of particles that can be achieved using the method of Applicants' invention. The distribution of particles formed using Applicants' seeded citrate method, a malate reduction method and hydroxylamine seeded particles are shown. The narrowest distribution was achieved with seeded citrate, the next narrowest with seeded hydroxylamine, and the broadest with malate as the reducing agent.

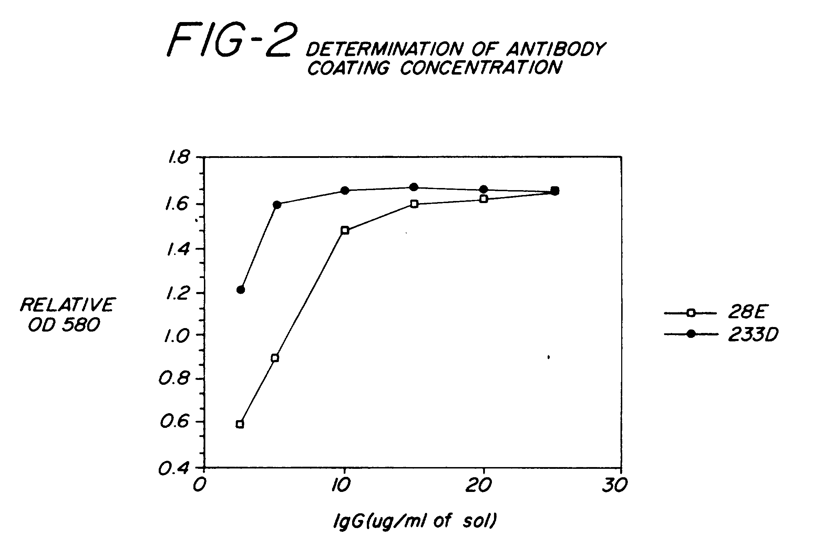

Figure 2 is a graph which shows the determination of the antibody coating concentration for a sol made in accordance with the teachings of Applicants' invention.

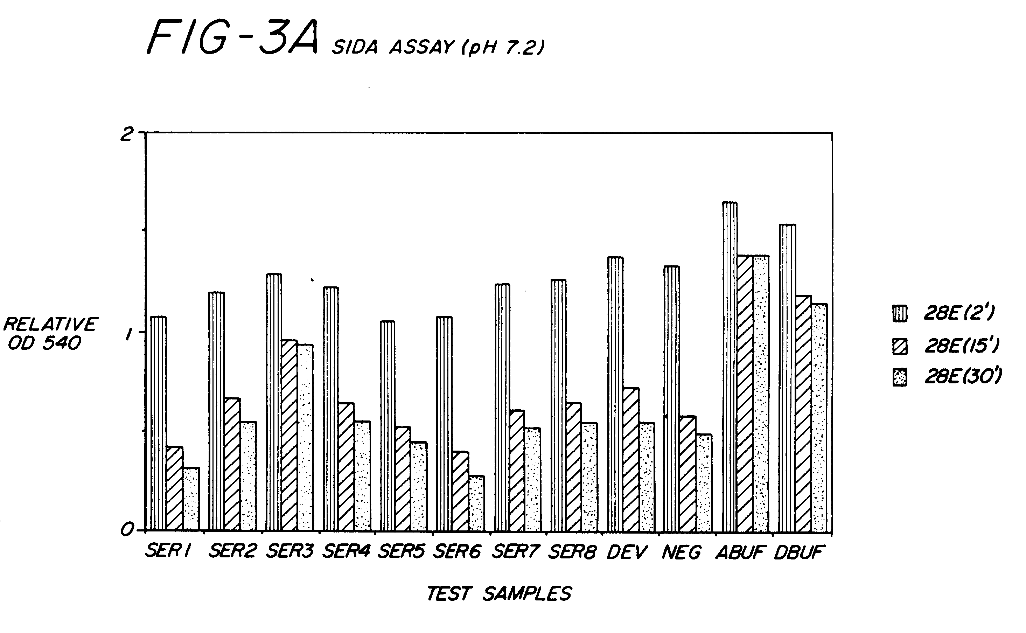

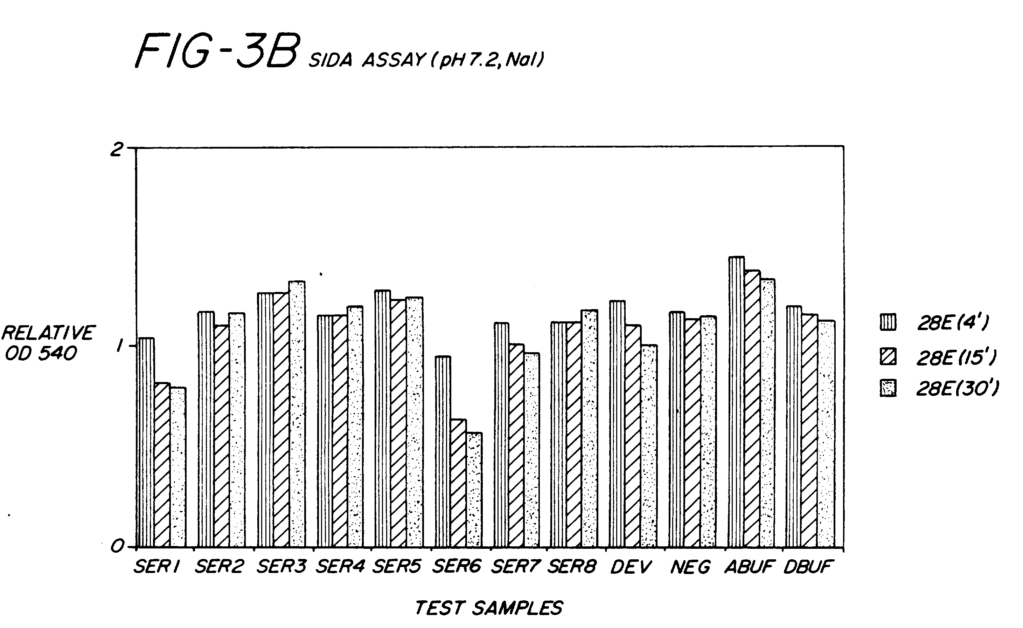

Figure 3 is a bar graph which illustrates the effect of adding a chaotrophic agent to the assay buffer. For the clones evaluated, the signal was significantly improved by adding the agent when Figure 3A (without the addition of chaotrophic agent) is compared with Figure 3B (with the chaotrophic agent added), showing that serum induced aggregation rates were reduced when these agents are used.

### Detailed Description of the Invention

Applicants' novel colloidal seeded metal sol process produces a reagent which can be utilized in microscopic and immunoassay techniques. Applicants first prepare nuclei or seed using a strong reducing agent such as sodium borohydride, hydrogen peroxide, ascorbic acid, phosphorus, sodium citrate or malic acid, in the presence of a stabilizing agent such as citrate, malate, citric acid, malic acid, oxalate, oxalic acid, aspartate, aspartic acid, glutamate, or glutamic acid. The reducing and stabilizing agent are selected so that they will not appreciably affect the subsequent growth of the nuclei, i.e., neither will generate spontaneous nuclei nor retard the growth of the nuclei during subsequent particle growth. Applicants' method for producing nuclei comprises adding a suitable stabilizing agent to a metal salt solution and then adding a suitable reducing agent. Preferably, a gold salt solution is used and most preferably a chloro-auro-tetrahydrate solution. The seed produced according to Applicants' method are very stable over time as shown in the following Table 1.

**Table 1**

| **Hydroxylamine Seeded Sol Process Validation (90 ml reaction volume, 2x gold sol concentration)** | | | | |
|---|---|---|---|---|
| Seed Lot # (a) | Average Particle Size (nm) | | | Date (b) |
| Seed Volume (ul) | 2 | 3 | 4 | |
| 100 | 55 | | 48 , 47 | 10/22/87 |
| | 56 | 56 | 52 , 61 | 11/16/87 |
| | 55 | 55 | 52 , 52 | 12/08/87 |
| | 57 | 58 | 54 | 03/21/88 |
| | | | | |
| 60 | 65 | | 57 , 55 | 10/22/87 |
| | 66 | 67 | 61 , 60 | 11/16/87 |
| | 64 | 68 | 61 , 60 | 12/08/87 |
| | 66 | 70 | 64 | 03/21/88 |
| 20 | 92 | | 79 , 78 | 10/22/87 |
| | 102 | 96 | 86 , 87 | 11/16/87 |
| | 93 | 96 | 86 , 89 | 12/08/87 |
| | 94 | 99 | 90 | 03/21/88 |

| | | | | |
|---|---|---|---|---|
| Note (a) Seed lot numbers 2, 3 and 4 were prepared on 08/27/87, 08/31/87 and 10/22/87, respectively. | | | | |
| Note (b) Dates the gold sol were prepared. | | | | |

The nuclei produced are then added to a metal-containing solution, preferably a metal salt solution, containing a second reducing agent which retards spontaneous enucleation and allows metal sol particles to form. Hydroxylamine is the most preferable reducing agent for this step. The reaction is nearly independent of the concentration of the reducing agent, as shown in the following Table 2.

**Table 2**

| **Effect of Hydroxylamine Concentration on Particle Size and Particle Distribution** | | |
|---|---|---|
| Hydroxylamine (50 mg/ml) | Particle Size (nm) | Particle Peak Width |
| 0.4 ml | 63.3 | 47.87 |
| 0.2 ml | 63.3 | 46.89 |
| 0.08 ml | 66.4 | 47.97 |

Other useful reducing agents which retard spontaneous enucleation include substituted hydroxylamine, hydrazine, and substituted hydrazine. This step can also be performed by using separate reducing and enucleation retarding agents.

The reducing agent is then inactivated. This can be achieved by pH adjustment, a combination of pH and bovine serum albumin, or by using a chemical agent. Ketones, aldehydes, amino acids or various combinations of chemical agents, e.g., ascorbic acid, ascorbic acid and acetone, ascorbic acid and fructose, are effective inactivators. Other useful chemical inactivators include reducing agents such as a hydride or hydrogen and oxidizing agents such as oxygen or a peroxide.

In another embodiment of this invention, a reducing agent is added before a significant amount of spontaneous enucleation occurs. Reducing agents which are useful in this embodiment include citrate, malate, malic acid, tannate, tannic acid, hydrogen peroxide, oxalate, glutamate, stannous chloride, and sodium hydroxide. To prepare a citrate seeded gold sol, sodium citrate is added to a chloroauric acid solution, followed immediately by the addition of sodium borohydride prepared seed. The solution turns from a faintly blue to a dark red within less than a minute. Neither prolonged heating or the addition of extra citrate or seed produces any substantial change in the solution after that period. As shown in Figure 1, this method produces particles of a selected size and narrow distribution.

The metal sol particles formed are then coated with a selected material. The particles can be coated with a proteinaceous material such as an antibody or an antigen, or conjugates thereof, for example. They may also be coated with polysaccharides, lectins, peptides, conjugated peptides or surfactants, as appropriate for the use of the reagent.

When the coating antibody is pretreated with a reducing agent and capped with a sulfhydryl blocking agent, it was surprisingly found that the resulting derivatized antibody, when coated on gold sol, exhibits an improved signal generation. Useful reducing agents include dithiothreitol, dithioerythritol, glutathione, cysteine, β-mercaptoethanol, sodium borohydride, borohydride salts. Useful blocking agents include iodoacetamide, iodoacetic acid, iodoacetate salts and N-ethyl maleimide.

The coated particles are then post-coated by coating the particles with delipidated milk, most preferably non-fat dry milk which Applicants surprisingly found to be a very effective post-coater. The post-coated particles are then stored in a buffer solution containing albumin, most preferably bovine serum albumin, and a low molecular weight sugar, most preferably, mannitol.

Regardless of the reactor vessel shape (which can range from a 17x100 mm polypropylene tube to a 1000 ml Erlenmeyer flask or spherical reaction flask or 50,000 ml cylindrical reaction flask) the same particle size sol can be prepared at a bench top scale of 4.5 ml and proportionally scaled up to 40,000 ml according to the teachings of Applicants' method. In addition, the same procedure will yield sol with the same particle size for 1x fold sol up to 10x sol. The effect of gold concentration on particle size is shown in the following Table 3.

**Table 3**

| **Effect of Gold Concentration on Particle Size** | | |
|---|---|---|
| Volume of 2% Au Added (ml) | Particle Size (nm) | Change in Avg. Particle Size (nm) |
| 0.8 (-20%) | 58.7 | -3.9 |
| 0.9 (-10%) | 60.6 | -2 |
| 1 | 62.6 | 0 |
| 1.1 (+10%) | 65.6 | 3 |
| 1.2 (+20%) | 66.4 | 3.8 |

In a kit for performing an immunoassay comprising a reagent made in accordance with Applicants' method and an assay buffer containing a chaotrophic agent it was surprisingly found that serum induced aggregation can be eliminated or minimized when a chaotrophic agent is added to the assay buffer. The chaotrophic agent can be comprised of a combination of an ion selected from the group consisting of NO₃⁻, ClO₄⁻, I⁻, SCN⁻, Na⁺, Cs⁺, Li⁺, Mg²⁺, Ca²- and Ba²⁺, most preferably NaI. The addition of 1mM NaI to an optimized HBsAg assay buffer virtually eliminated this problem for HBsAg clones as shown in Figure 3. In the absence of NaI, serum induced aggregation of the reagents is evident within minutes. After 30 minutes at room temperature, colloidal gold reagents can be seen forming large aggregates depositing at the bottom of the test tubes or microwells. Although other non-chaotrophic agents such as dextran sulfate, reduction of the KCl or NaCl concentration in the assay buffer, or high temperature (up to 40°C) alleviate the serum induced aggregation problem to some extent, none are as effective as NaI. Other chaotrophic agents useful in Applicants' invention include guanidine, urea, TCA and TFA.

A rapid, simple and qualitative dot blot assay is also provided to determine the presence and/or immunological activity on coated metal sol particles. The dot blot assay is a membrane filtration type assay used to qualitatively detect the presence of IgG, TSH or HCG, for example, adsorbed onto the gold particles. For example, a gold reagent made in accordance with Applicants' method appropriately diluted in a buffer containing Tween 20 is filtered through a nitrocellulose membrane onto which goat anti-mouse IgG is immobilized. Mouse IgG coated gold particles will be retained on the filter as a reddish dot (within seconds) whereas unreacted materials will be readily washed away. Alternatively this method can be used as an immunoassay tool to detect the presence of HCG or TSH in serum samples. The assay involves the immobilization of a mouse anti-HCG or anti-TSH to the nitrocellulose filter paper. A mixture containing sample and anti-TSH or HCG gold reagent in an appropriate buffer is then filtered through the nitrocellulose membrane. TSH and HCG can be readily detected in Lyphocheck Immunoassay Control Serum Levels I within 15 seconds.

The invention is further illustrated by means of the following examples. These examples are meant to be illustrations only and are not intended to limit the present invention to these specific embodiments.

### Example 1

### Particle Size Data

A DuPont sedimentation of field flow fractionator (SF3) was used to determine gold sol size distributions. This instrument resembles an HPLC instrument with a thin rotating channel, mounted in a centrifuge, replacing the column. A UV detector quantitates the particles by absorption and feeds data to a computer/controller for size computation. The principle of separation is based on the fact that large dense particles are pushed closer to the wall of the chromatography channel than small particles when a centripetal force is applied. This instrument was calibrated with gold particles against electron microscopy and found to be far superior to light scatter correlation instruments for gold sols of this size. The resolution of the instrument is approximately one nanometer.

The peak diameters for 30 samples are summarized in Table 4.

**Table 4**

| **EFFECT OF REACTOR VOLUME, SOL CONCENTRATION AND SEED VOLUME ON THE GOLD SOL AVERAGE PARTICLE SIZE** | | | |
|---|---|---|---|
| | Particle Size (nm) | Reactor Volume (ml) | BOL Concentration |
| 09/28/87 | 68.8 | 90 | 1x |
| | 66.4 | 90 | 1x |
| | 76.7 | 90 | 1x |
| | 69.7 | 450 | 1x |
| | 92.9 * | 90 | 1x |
| | 114.0 ** | 90 | 1x |
| 10/01/87 | 75.7 | 90 | 1x |
| | 75.7 | 450 | 1x |
| 10/02/87 | 74.5 | 450 | 1x |
| | 67.5 | 450 | 1x |
| | 68.5 | 450 | 1x |
| 10/05/87 | 68.5 | 4.5 | 8x |
| | 68.5 | 4.5 | 8x |
| 10/06/87 | 70.5 | 4.5 | 8x |
| | 69.7 | 4.5 | 10x |
| | 81.8 *** | 4.5 | 10x |
| | 62.3 **** | 4.5 | 10x |
| 10/13/87 | 74.3 | 450 | 1x |
| | 67.5 | 450 | 1x |
| | 68.6 | 450 | 1x |
| 11/01/87 | 72.0 | 450 | 1x |
| 11/10/87 | 76.0 | 4050 | 1x |
| 11/13/87 | 72.0 | 450 | 1x |
| 12/09/87 | 71.0 | 900 | 1x |
| 12/21/87 | 75.0 | 450 | 1x |
| 12/22/87 | 75.0 | 1800 | 1x |
| 01/11/88 | 73.0 | 450 | 1x |
| | 73.0 | 900 | 1x |
| 01/12/88 | 75.0 | 450 | 1x |
| 01/28/88 | 69.7 | 40000 | 1x |
| Unless specified otherwise, the seed volume used was proportionally scaled to give 70 nm particles, .02 ml for 90 ml 1x sol. | | | |

| | | | |
|---|---|---|---|
| * The seed volume was 0.010 ml | | | |
| ** The seed volume was 0.005 ml | | | |
| *** The seed volume was 0.005 ml | | | |
| **** The seed volume was 0.015 ml | | | |

### Example 2

### Preparation of Sodium Borohydride Seed Nuclei

To 900 ml of water purified by MilliQ or a similar system, 10 ml of 1% chloroauric acid is added. The mixture is stirred for 1 minute at room temperature. 10 ml of 1% sodium citrate is added. The mixture is stirred for 1 minute at room temperature. 10 ml of fresh 0.075% sodium borohydride dissolved in 1% sodium citrate is added. The mixture is stirred for 5 minutes at room temperature, then sterile filtered and stored at 4°C.

### Example 3

### Preparation of Hydroxylamine Seeded Gold Sol

To 900 ml of MilliQ water, 10 ml of 2% chloroauric acid is added at room temperature under continuous stirring. 4 ml of fresh 50 mg/ml hydroxylamine chloride is added. 0.4 ml of sodium borohydride seed, prepared as described in Example 2, are added. The mixture is stirred continuously for 5 minutes at room temperature. This procedure yields 70 nm particles.

### Example 4

### Preparation of Citrate Seeded Sol

The standard procedure for obtaining citrate seeded sol is as follows. To 450 ml of MilliQ water heated to 95°C was added 5 ml of 1% HAuCl4 with constant stirring. When the temperature was re-equilibrated to 95°C, 2 ml of 1% Na-Citrate was added followed immediately by 0.15 ml of NaBH4 seed. The reaction was then allowed to proceed for another 15 minutes at 95°C. The solution turned first from a faint blue rapidly to dark red within 60 seconds. Neither prolonged heating or the addition of extra citrate or seed produced any substantially change in the solution after that period.

### Example 5

### Gold Sol Pretreatment for Coating pH and Coating Concentration Determination

The gold sol pH is adjusted to pH 6.5 with 0.2 M potassium carbonate. Bovine serum albumin is added to a final concentration of 2 mg/ml of sol and stirred for 30 minutes.

### Example 6

### Gold Sol Pretreatment for Reagent Preparation

The gold sol pH is adjusted to the desired coating pH or to pH 7.0 if the coating pH is above 7.0. BSA is added to a final concentration of 2 mg/ml of sol and stirred for 30 minutes.

### Example 7

### Reagent Coating Procedures

### A. Coating pH Determination

The protein was diluted in an appropriate Good's buffer to 250 mg/ml and 100 µl was added to each glass or polypropylene tube. Sigma Chemical Company's publication concerning buffers sold by that company lists a number of Good's buffers and their compositions which would be useful in Applicants' invention. The gold sol was titrated to pH 6.0 to 9.0 in 1.0 pH increments with fresh .02 M potassium carbonate removing 1 ml at each point and adding each aliquot to the protein solution. The mixtures were allowed to stand for 30 minutes at room temperature. 100 ml of a 10% sodium chloride solution was added and allowed to stand for 10 minutes at room temperature. Using water as a blank, the absorbance at 580 nm was read using a spectrophotometer and recorded for each sample. The results, shown in Figure 1, are plotted using absorbance as the Y axis and pH as the X axis. The coating pH is that at which the absorbance readings level off and become parallel to the X axis. This reading will be the same as the control sample in which 100 µl at buffer is added instead of the 10% sodium chloride.

### B. Coating Concentration Determination

The coating protein is diluted to 25, 50, 100, 150, 200 and 250 mg/ml in an appropriate buffer determined in part A and 100 ml added to each tube. The gold sol is titrated to the pH determined in A, above, with 0.2 M potassium carbonate. 1.0 ml of pH adjusted gold sol is added to each tube and allowed to stand for 30 minutes at room temperature. 100 ml of 10% sodium chloride is added to each tube and allowed to stand 10 minutes at room temperature. Using water as a blank, the absorbance is recorded at 580 nm for each sample. The absorbance versus mg protein/ml of sol is then plotted. The coating level is that just before the absorbance levels off. See Figure 1.

### C. Coating Procedure

The gold sol is titrated to the coating pH with 0.2 M potassium carbonate. The required amount of protein is diluted to 1/20th the sol volume in 10 mM of the appropriate coating buffer. Alternatively, the protein is diluted in the appropriate coating buffer containing 0.002% BSA. The protein solution is added to the raw sol and allowed to stand for a minimum of 60 minutes at room temperature. The sol is then post-coated with 1/10th sol volume of a 0.1% non-fat dry milk in water solution, which has been 0.45 µm filtered for 30 minutes (final milk concentration is 0.01%). The coated sol is washed by centrifuging and removing the supernatant. The sol is resuspended in a 10 mM Good's buffer 0.5% BSA (RIA grade), 300 mM mannitol, 0.01% sodium azide, pH 7.0 (storage buffer). The washing step is performed three times. After the third centrifugation, storage buffer is added to 1/10th initial sol volume (i.e., 100 ml for each 1000 ml of raw sol). The absorbance is read at 540 nm. The volume is adjusted such that the final absorbance is 10 OD units. The solution is then filtered through a 0.2 µm low protein-binding filter and stored.

### Example 8

### Hepatitis Virus Surface Antigen Test

Polycarbonate cuvettes were coated with mouse monoclonal anti-hepatitis surface antigen antibodies by incubating 200 microliters of a 100 microgram per ml solution of antibody in 0.01 M phosphate buffered saline, pH 7.4, overnight at room temperature followed by three aspirate fill steps with 300 microliters of 0.05 M Hepes/Tris, pH 8.3 buffer. The cuvettes were then overcoated with 300 microliters of 0.05 M Hepes/Tris, ph 8.3, buffer containing 1% bovine serum albumin for 60 minutes at room temperature, washed twice with 300 microliters of overcoating solution, incubated 15 minutes at room temperature with 300 microliters of 3% trehalose in 0.05 M Hepes/Tris, pH 8.3 buffer, aspirated, dried in room air and stored at room temperature in a desiccator below 20% relative humidity. The cuvettes were then mounted in applicant's STIR instrument.

Seventy-two microliters of standard, prepared by adding the appropriate amount of hepatitis surface antigen (from Merck) to a negative serum pool, was dispensed into cuvettes by an automated pipette and allowed to incubate in the enclosed 37°c instrument for five minutes. The pipette then dispensed 54 microliters of buffer (containing 2.0 M potassium chloride, 2% bovine serum albumin, 50 micrograms per ml of normal mouse IgG and 0.05% sodium azide dissolved in 0.05 M sodium barbital buffer at pH 8.5) and 180 microliters of 105 nm diameter 0.1% monoclonal anti-hepatitis surface antigen coated gold colloid suspension (in 10 mM Hepes buffer, pH 7.0, containing 0.05% sodium azide, 300 mM mannitol, and 0.5% bovine serum albumin) at a rate sufficient to mix the fluids in the cuvette. The light scattered by each cuvette was then recorded for the next ten minutes. The time integral of the fifth order lineal regression curve fit of the light scatter versus time data was reported for each cuvette. The average of the signal from five of the six zero standards (one was 14 standard deviations from mean of the other five) and the average of the duplicate standards correlated proportionately with the hepatitis surface antigen present, as can be seen from the following data:

| **HEPATITIS SURFACE ANTIGEN CONCENTRATION** | **MEAN SIGNAL** |
|---|---|
| 0 | 1.7358 |
| 0.1 ng/ml | 2.2376 |
| 0.2 ng/ml | 2.9421 |
| 0.4 ng/ml | 3.99235 |
| 0.6 ng/ml | 5.0442 |
| 0.8 ng/ml | 6.72185 |
| 1.0 ng/ml | 7.0187 |
| 1.5 ng/ml | 9.31175 |
| 2.0 ng/ml | 10.7365 |
| 2.5 ng/ml | 14.0444 |
| 5.0 ng/ml | 24.9279 |
| 10.0 ng/ml | 47.4585 |

### Example 9

### Anti-Hepatitis Core Antigen Human Antibody Test

Polycarbonate cuvettes were coated as in Example 8, with recombinant hepatitis core antigen using a 5 microgram per ml coating solution. The cuvettes were then dried, stored and mounted in the STIR instrument, which was enclosed and equipped with 37° air circulation. Seventy-two microliters of the appropriate sample or control were added to separate cuvettes by an automated pipette and allowed to incubate for 5 minutes, after which time 54 microliters of assay buffer (consisting of 1% bovine serum albumin and 1 M NaCl dissolved in pH 7.4 phosphate buffered saline) and 180 microliters of a 0.1% suspension of 105 nanometer diameter mouse monoclonal anti-human IgG coated colloidal gold suspension was dispensed with sufficient velocity to mix the contents of the cuvette. The light scattered by the cuvette was then recorded for the next 10 minutes. The time integral of the fifth order linear regression curve fit of the scattered light versus time data was reported for each cuvette as signal. The mean signal of replicates of each serum sample correlated with the presence of anti-hepatitis core antigen employing a cutoff of 3 standard deviations above the mean of the negative control, as show in the following data:

| **Sample Type** | **Number of Replicates** | **Mean** | **Test Outcome** |
|---|---|---|---|
| Negative control | 4 | 0.9524 | S.D.=0.51 |
| Positive control | 4 | 60.2099 | S.D.=6.5 |
| Negative sample 1 | 2 | 2.1671 | - |
| Positive sample 1 | 2 | 10.483 | + |
| Positive sample 2 | 2 | 41.058 | + |
| Positive sample 3 | 2 | 33.494 | + |
| Positive sample 4 | 2 | 2.6043 | + |
| Positive sample 5 | 2 | 74.2235 | + |

### Example 10

### Example of TSH STIR Assay With Hydroxylamine Seeded particles

Gold particles of 77.7 nm diameter were prepared by adding seed particles to a gold salt-hydroxylamine mixture as described above and coated by the standard method with anti-TSH monoclonal antibody. 36 microliters of Cone Biotech commercial standard plus 128 microliters of assay buffer plus 80 microliters coated gold sol were incubated for 5 minutes at 37°C in a rotating parabolic mirror automatic STIR instrument. After incubation a 150 microliter aliquot was delivered to an anti-TSH monoclonal coated STIR cuvette and read for 10 minutes. The assay buffer contained 0.05 M Barbital, 1 M KCl, 1% BSA, .05% NaN₃ and 100 micrograms per ml of polymerized mouse IgG, adjusted to pH 8.5. The polymerized mouse IgG was found to be the optimum agent to eliminate anti-mouse activity caused nonspecific reactions in STIR assays. The integral of the fifth order fit of the measured scattered light, the STIR signal, was as follows:

| **Duplicate Signal of TSH Standards** | | | | |
|---|---|---|---|---|
| Zero STD | 0.5µIU/ml | 2µIU/ml | 5µIU/ml | 20µIU/ml |
| 0.419 | 3.624 | 13.44 | 34.04 | 140.2 |
| 0.321 | 3.714 | 12.80 | 33.37 | 143.8 |

### Example 11

### Reduction/Blocking Pretreatment of Coating Antibody to Enhance Signal Generation

The antibody is reacted with an appropriate reducing agent, e.g., DTT, β-mercaptoethanol, at a concentration of 0.01 to 0.1 M for at least 5 minutes at a temperature of 2°C to 40°C. After a sufficient time period, an appropriate sulfhydryl blocking agent is added, e.g., iodoacetamide, N-ethyl-maleimide, at a concentration of 0.1 M to 1.0 M for at least 1 minute. The material is then desalted by dialysis or gel filtration.

In one experiment, 0.01 M DTT is reacted with the antibody of interest for 30 minutes at room temperature, followed by reaction with 0.1 M iodoacetamide for 5 minutes, at room temperature. The material is then dialyzed into 5 mM MOPSO.

### Example 12

### Preparation of Reduced/Blocked Antibody Coated Gold

To 1 ml of mouse anti-hCG monoclonal antibody (clone number 16a) at 17.6 mg/ml in PBS with 0.1% sodium azide is added 15.4 mg solid dithiothreitol (0.1 M) at 22°C. The mixture is incubated for 30 min. After incubation, 185 mg solid iodoacetamide (1.0 M) is added and allowed to react for 5 minutes. The antibody is separated from the reactants by dialysis versus 5 mM MOPSO, pH 7.0 and coated in the usual fashion. When coated on gold sol, it was found that the derivatized antibody exhibits an improved signal generation as shown in the following data:

| Sample | Net Signal | Signal 0 µIU/ml | Signal 100 µIU/ml |
|---|---|---|---|
| standard reagent | 395.32 | 3.68 | 399 |
| reduced/blocked reagent | 550.50 | 5.50 | 556 |

As seen from the above, a simple reproducible method for producing a gold reagent containing a narrow distribution of colloidal gold particles of a selected size is provided.

While the foregoing description has been directed to the preferred embodiments of the present invention, those of ordinary skill in the art in this field will appreciate that various modifications can be made in the materials and methods described herein without departing from the scope of the present invention, which is described more particularly in the claims appended hereto.

### Example 13

### Metal Coated Latex Particles

A small volume of sodium borohydride seed nuclei, prepared as described in Example 2, are added to a dilute suspension of polymer latex particles to allow attachment of the seed nuclei to the latex particles' surface. 2% chloroauric acid and hydroxylamine hydrochloride, 50 mg/ml, are then added to the mixture stirred continuously for 5 minutes at room temperature. This procedure produces polymeric latex particles coated with metallic gold capable of being coated as described in the foregoing examples. These particles exhibit settling rates intermediate between solid gold particles and uncoated latex particles. An advantage of this method is that slow settling, inexpensive very large particles (e.g., microns in diameter) can be prepared without the rapid settling problems of large solid gold particles.

## Claims

1. A method for producing metallic sol particles of a selected size range which comprises:
(a) reducing a solution containing a metal and a stabilising agent with a first reducing agent to form metallic nuclei; and
(b) mixing the metallic nuclei formed in step (a) with a solution containing said metal and a second reducing agent to form metallic sol particles,
wherein:
said stabilising agent is citrate, malate, citric acid, malic acid, oxalate, oxalic acid, aspartate, aspartic acid, glutamate or glutamic acid; and said stabilizing agent are
said first reducing agent selected so that in step (b) they will not generate nuclei and will not retard growth of nuclei in the subsequent particle growth step; and
said second reducing agent retards spontaneous enucleation.

2. The method of claim 1, wherein said first reducing agent is a borohydride salt, hydrogen peroxide, ascorbic acid, phosphorous, sodium citrate or malic acid.

3. The method of claim 2, wherein said first reducing agent is a borohydride salt, preferably sodium borohydride.

4. The method of any one of claims 1 to 3, wherein said second reducing agent is hydroxylamine, hydrazine, substituted hydroxylamine or substituted hydrazine.

5. The method of claim 4, wherein said second reducing agent is hydroxylamine or hydroxylamine hydrochloride.

6. The method of any one of claims 1 to 5, wherein the stabilising agent is sodium citrate.

7. The method of any one of claims 1 to 6, wherein step (a) comprises adding said stabilising agent to said metal-containing solution and then adding said first reducing agent to the stabilised metal-containing solution.

8. The method of any one of claims 1 to 7, wherein between steps (a) and (b), the metallic nuclei are absorbed onto a polymeric surface.

9. The method of any one of claims 1 to 8, wherein step (b) is carried out to produce sol particles having a diameter from 10 to 200 nm.

10. The method of any one of claims 1 to 9, further including the step of inactivating any reactive species present after the completion of step (b).

11. The method of claim 10, wherein the inactivation step is carried out by adjusting the pH of the sol.

12. The method of claim 10 or claim 11, wherein the inactivation step is carried out by adding a chemical agent.

13. The method of claim 12, wherein the chemical agent is a proteinaceous agent, such as albumin, preferably bovine serum albumin.

14. The method of any one of claims 10 to 13, further including the step of coating the inactivated sol particles with a selected, preferably proteinaceous, material.

15. The method of claim 14, wherein the selected material is an antibody.

16. The method of claim 15 wherein the antibody has been reduced using a third reducing agent, such as dithiothreitol, dithioerythritol, glutathione, cysteine, β-mercaptoethanol, or a borohydride salt, and capped with a sulfhydryl blocking agent, such as iodoacetamide, iodoacetic acid, an iodoacetate salt or N-ethylmaleimide.

17. The method of any one of claims 14 to 16, further including the step of post-coating sol particles, preferably with a delipidated milk such as non-fat dry milk.

18. The method of any one of claims 14 to 17, further including the step of adding said coated or post-coated sol particles to a buffer solution comprising an albumin, preferably bovine serum albumin, and a low molecular weight sugar, preferably mannitol.

19. The method of any one of claims 1 to 18, wherein the metal-containing solutions contain gold, preferably in the form of a gold salt such as chloro-auro-tetrahydrate.

20. A kit for performing an immunoassay comprising: coated or post-coated sol particles produced by the method of any one of claims 14 to 18; and an assay buffer.

21. The kit of claim 20, wherein said assay buffer contains a chaotropic agent, which is preferably an NO₂⁻, ClO₄⁻ I⁻, SCN⁻, Na⁺, Cs⁺, Li⁺, Mg²⁺, Ca²⁺ or Ba²⁺ ion, guanidine, urea, TCA or TFA, and is more preferably NaI.

## Patentansprüche

1. Ein Verfahren zum Herstellen von metallischen Solpartikeln mit einem ausgewählten Größenbereich, das umfaßt:
a) Reduzieren einer ein Metall und ein Stabilisierungsmittel enthaltenden Lösung mit einem ersten Reduktionsmittel, um metallische Nuklei zu bilden; und
b) Mischen der in Schritt a) gebildeten metallischen Nuklei mit einer Lösung, die das Metall und ein zweites Reduktionsmittel enthält, um metallische Solpartikel zu bilden,
worin
das Stabilisierungsmittel Citrat, Malat, Zitronensäure, Äpfelsäure, Oxalat, Oxalsäure, Aspartat, Asparaginsäure, Glutamat oder Glutaminsäure ist;
das erste Reduktionsmittel und das Stabilisierungsmittel so ausgewählt sind, daß sie in Schritt (b) keine spontanen Nuklei erzeugen werden und Wachstum von Nuklei in dem nachfolgenden Partikelwachstumsschritt nicht verzögern werden; und
das zweite Reduktionsmittel spontane Enukleation verzögert.

2. Das Verfahren nach Anspruch 1, wobei das erste Reduktionsmittel ein Hydridoboratsalz, Wasserstoffperoxyd, Ascorbinsäure, Phosphor, Natriumcitrat oder Äpfelsäure ist.

3. Das Verfahren nach Anspruch 2, wobei das erste Reduktionsmittel ein Hydridoboratsalz, vorzugsweise Natriumborhydrid, ist.

4. Das Verfahren nach einem der Ansprüche 1 bis 3, wobei das zweite Reduktionsmittel Hydroxylamin, Hydrazin, substituiertes Hydroxylamin oder substituiertes Hydrazin ist.

5. Das Verfahren nach Anspruch 4, wobei das zweite Reduktionsmittel Hydroxylamin oder Hydroxylaminhydrochlorid ist.

6. Das Verfahren nach einem der Ansprüche 1 bis 5, wobei das Stabilisierungsmittel Natriumcitrat ist.

7. Das Verfahren nach einem der Ansprüche 1 bis 6, wobei Schritt (a) umfaßt Zugeben des Stabilisierungsmittels zu der Metall enthaltenden Lösung und dann Zugeben des ersten Reduktionsmittels zu der stabilisierten Metall enthaltenden Lösung.

8. Das Verfahren nach einem der Ansprüche 1 bis 7, wobei zwischen den Schritten (a) und (b) die metallischen Nuklei auf eine Polymeroberfläche absorbiert werden.

9. Das Verfahren nach einem der Ansprüche 1 bis 8, wobei Schritt (b) ausgeführt wird, um Solpartikel mit einem Durchmesser von 10 bis 200 nm herzustellen.

10. Das Verfahren nach einem der Ansprüche 1 bis 9, das weiter den Schritt umfaßt, irgendwelche nach Abschluß von Schritt (b) vorhandenen reaktiven Spezien zu inaktivieren.

11. Das Verfahren nach Anspruch 10, wobei der Inaktivierungsschritt durchgeführt wird durch Einstellen des pH des Sols.

12. Das Verfahren nach Anspruch 10 oder 11, wobei der Inaktivierungsschritt durchgeführt wird durch Hinzugeben eines chemischen Agens.

13. Das Verfahren nach Anspruch 12, wobei das chemische Agens ein proteinhaltiges Agens, wie Albumin, bevorzugterweise Rinderserumalbumin, ist.

14. Das Verfahren nach einem der Ansprüche 10 bis 13, welches weiter den Schritt umfaßt, die inaktivierten Solpartikel mit einem ausgewählten, vorzugsweise proteinhaltigen, Material zu beschichten.

15. Das Verfahren nach Anspruch 14, wobei das ausgewählte Material ein Antikörper ist.

16. Das Verfahren nach Anspruch 15, wobei der Antikörper unter Verwendung eines dritten Reduktionsmittels, wie Dithiothreitol, Dithioerythritol, Glutathion, Cystein, β-Mercaptoethanol oder eines Hydridoboratsalzes reduziert worden ist und mit einem Sulfhydrylblockierungsagens, wie Jodacetamid, Jodessigsäure, einem Jodacetatsalz oder N-Ethylmaleimid abgedeckt worden ist.

17. Das Verfahren nach einem der Ansprüche 14 bis 16, welches weiter den Schritt umfaßt, Solpartikel nachträglich zu beschichten, bevorzugterweise mit einer entfetteten Milch, wie fettfreier Trockenmilch.

18. Das Verfahren nach einem der Ansprüche 14 bis 17, welches weiter den Schritt umfaßt, die beschichteten oder nachbeschichteten Solpartikel zu einer Pufferlösung hinzuzugeben, die ein Albumin, bevorzugterweise Rinderserumalbumin, und einen niedermolekularen Zucker, vorzugsweise Mannitol, enthält.

19. Das Verfahren nach einem der Ansprüche 1 bis 18, wobei die Metall enthaltenden Lösungen Gold, bevorzugterweise in der Form eines Goldsalzes, wie Chlor-Gold-Tetrahydrat, enthalten.

20. Ein Kit zur Durchführung eines Immunoassays, der umfaßt: Beschichtete oder nachbeschichtete Solpartikel, die mittels des Verfahrens nach einem der Ansprüche 14 bis 18 hergestellt sind; und einen Testpuffer.

21. Der Kit nach Anspruch 20, wobei der Assaypuffer ein chaotropes Mittel enthält, welches bevorzugterweise ein NO₂⁻-, ClO₄⁻ -, I⁻-, SCN⁻-, Na⁺-, Cs⁺-, Li⁺-, Mg²⁺-, Ca²⁺-, oder Ba²⁺-Ion, Guanidin, Harnstoff, TCA oder TFA ist und bevorzugterweise NaI.

## Revendications

1. Méthode de production de particules d'un sol métallique d'une gamme sélectionnée de grandeurs qui comprend :
(a) la réduction d'une solution contenant un métal et un agent stabilisant avec un premier agent réducteur pour former des noyaux métalliques ; et
(b) le mélange de noyaux métalliques formés à l'étape (a) avec une solution contenant ledit métal et un second agent réducteur pour former des particules d'un sol métallique,
où :
ledit agent stabilisant est citrate, malate, acide citrique, acide malique, oxalate, acide oxalique, aspartate, acide aspartique, glutamate ou acide glutamique ; et
ledit premier agent réducteur et ledit agent stabilisant sont sélectionnés de manière qu'à l'étape (b) ils ne produisent pas des noyaux spontanés et ne retardent pas la croissance des noyaux dans l'étape subséquente de croissance des particules ; et
ledit second agent réducteur retarde la nucléation spontanée.

2. Méthode de la revendication 1, où ledit premier agent réducteur est un sel de borohydrure, du peroxyde d'hydrogène, de l'acide ascorbique, du phosphore, du citrate de sodium ou de l'acide malique.

3. Méthode de la revendication 2, où ledit premier agent réducteur est un sel de borohydrure, de préférence le borohydrure de sodium.

4. Méthode selon l'une quelconque des revendications 1 à 3, où ledit second agent réducteur est l'hydroxylamine, l'hydrazine, l'hydroxylamine substituée ou l'hydrazine substituée.

5. Méthode de la revendication 4, où ledit second agent réducteur est l'hydroxylamine ou le chlorhydrate d'hydroxylamine.

6. Méthode selon l'une quelconque des revendications 1 à 5, où l'agent stabilisant est le citrate de sodium.

7. Méthode selon l'une quelconque des revendications 1 à 6, où l'étape (a) comprend l'addition dudit agent stabilisant à ladite solution contenant un métal puis l'addition dudit premier agent réducteur à la solution stabilisée contenant un métal.

8. Méthode selon l'une quelconque des revendications 1 à 7, où entre les étapes (a) et (b), les noyaux métalliques sont absorbés sur une surface polymérique.

9. Méthode selon l'une quelconque des revendications 1 à 8, où l'étape (b) est effectuée pour produire des particules de sol ayant un diamètre de 10 à 200 nm.

10. Méthode selon l'une quelconque des revendications 1 à 9, comprenant de plus l'étape d'inactiver toute espèce réactive présente après accomplissement de l'étape (b).

11. Méthode de la revendication 10, où l'étape d'inactivation est effectuée en ajustant le pH du sol.

12. Méthode de la revendication 10 ou de la revendication 11, où l'étape d'inactivation est effectuée en ajoutant un agent chimique.

13. Méthode de la revendication 12, où l'agent chimique est un agent protéiné comme l'albumine, de préférence l'albumine de sérum bovin.

14. Méthode selon l'une quelconque des revendications 10 à 13, comprenant de plus l'étape d'enduire les particules de sol inactivé avec une matière sélectionnée, de préférence protéinée.

15. Méthode de la revendication 14, où la matière sélectionnée est un anticorps.

16. Méthode de la revendication 15, où l'anticorps a été réduit en utilisant un troisième agent réducteur tel que le dithiothréitol, le dithioérythritol, la glutathione, la cystéine, le β-mercaptoéthanol, ou un sel de borohydrure, et coiffé avec un agent bloquant de sulfhydryle comme un iodoacétamide, de l'acide iodoacétique, un sel d'iodoacétate ou du N-éthylmaléimide.

17. Méthode selon l'une quelconque des revendications 14 à 16, comprenant de plus l'étape de post-enduire les particules de sol, de préférence avec un lait délipidé tel qu'un lait sec écrémé.

18. Méthode selon l'une quelconque des revendications 14 à 17, comprenant de plus l'étape d'ajouter lesdites particules de sol enduites ou post-enduites à une solution tampon comprenant une albumine, de préférence de l'albumine de sérum bovin et un sucre de faible poids moléculaire, de préférence du mannitol.

19. Méthode selon l'une quelconque des revendications 1 à 18, où les solutions contenant du métal contiennent de l'or, de préférence sous la forme d'un sel d'or tel que le chloro-auro-tétrahydrate.

20. Trousse pour accomplir une immunodétermination comprenant : des particules d'un sol enduites ou post-enduites, produites par la méthode selon l'une quelconque des revendications 14 à 18 ; et un tampon d'essai.

21. Trousse de la revendication 20, où ledit tampon d'essai contient un agent chaotropique qui est de préférence un ion NO₂⁻, ClO₄⁻, I⁻, SCN⁻, Na⁺, Cs⁺, Li⁺, Mg²⁺, Ca²⁺ ou Ba²⁺, la guanidine, l'urée, TCA ou TFA, et tout à fait de préférence NaI.
